# EUROPÄISCHE PATENTSCHRIFT

(11) **EP 2 157 115 B1**
(45) Veröffentlichungstag und Bekanntmachung des Hinweises auf die Patenterteilung: **28.09.2011**
(21) Anmeldenummer: 09162048.4
(22) Anmeldetag: 05.06.2009
(51) Int. Cl.: C08G 63/78, C02F 1/04, C02F 1/44, C07C 29/80

(54) **Verfahren und Vorrichtung zur Rückgewinnung von Ethylenglykol bei der Polyethylenterephthalatherstellung**
Device and method for regaining ethylene glycol when producing polyethylene terephthalate
Procédé et dispositif de récupération d'éthylène glycol lors de la fabrication de polyéthylène téréphtalate

(30) Priorität: 18.08.2008 DE 102008044440
(43) Veröffentlichungstag der Anmeldung: 24.02.2010
(73) Patentinhaber: Lurgi Zimmer GmbH, 60295 Frankfurt am Main (DE)
(72) Erfinder: Reimann, Randolf Dr., 63755, Alzenau (DE); Ambrassat, Rolf, 60489, Frankfurt am Main (DE)
(74) Vertreter: Meyer-Dulheuer, Karl-Hermann

(56) Entgegenhaltungen:
- WO-A1-96/35654
- US-A- 4 758 650
- DATABASE WPI Week 197843 Thomson Scientific, London, GB; AN 1978-77178A XP002542695 & JP 53 106794 A (MITSUBISHI RAYON CO LTD) 18. September 1978 (1978-09-18)

## Beschreibung

Die Erfindung betrifft ein Verfahren und eine Anlage bzw. Vorrichtung zur Rückgewinnung von Ethylenglykol bei der Polyethylenterephthalat (PET)-Herstellung.

PET wird über eine Veresterungsreaktion der Carboxylgruppen der Terephthalsäure und der Hydroxylgruppen des Ethylenglykols hergestellt, wobei sich Wasser abgespaltet. Das durch die Veresterungsreaktion anfallende Prozessabwasser, welches neben Acetaldehyd u. A. beträchtliche Mengen an Ethylenglykol enthält, wird einer Rektifikation (Prozesskolonne) zwecks Rückgewinnung des Ethylenglykols unterworfen. Um den Verlust an Ethylenglykol gering zu halten, weisen Verfahren nach dem Stand der Technik in der Regel baulich anspruchspruchsvolle Rektifikationskolonnen mit einer Vielzahl an Böden oder Packungen auf. Für eine befriedigend gute Abtrennung der im Prozessabwasser enthaltenen Verunreinigungen ist ein relativ hohes Rückflussverhältnis notwendig, was einen erheblichen Energiebedarf bedeutet. Trotz des energetisch aufwändigen Trennverfahrens wird das entstehende teilgereinigte Prozessabwasser aufgrund seines Restgehalts an Acetaldehyd und anderer flüchtiger organischer Komponenten anschließend meist noch über einen Stripper geführt, bevor es einer Kläranlage zugeleitet wird. Im PET-Herstellungsprozess fallen als Nebenprodukt der Polykondensationsreaktion außerdem erhebliche Mengen an verunreinigtem Ethylenglykol (Spalt-Ethylenglykol, SEG) an. Das SEG stammt in erster Linie vom Vakuumsystem und dem Ethylenglykolkreislauf der Vorpolykondensation und enthält 2-Methyl-1,3-dioxolan (MDO), in welchem Ethylenglykol in Form eines Acetals gebunden ist. Die SEG-Prozessströme aus den verschiedenen Prozessschritten des PET-Herstellungsverfahrens werden gemeinsam mit dem Prozessabwasser einer Aufarbeitung in der Rektifikationskolonne (hier auch als "Prozesskolonne" oder "Kolonne" bezeichnet) unterworfen und dann dem PET-Herstellungsprozess wieder zugeleitet. Beim MDO handelt es sich um einen Leichtsieder, der in der Prozesskolonne nicht zurückgehalten werden kann, d.h. über Kopf die Prozesskolonne verlässt. Der Großteil des MDO wird dann kondensiert und gelangt als Verunreinigung ins Prozessabwasser. Ein geringer Teil des MDO wird über die Abluft abgegeben. Das MDO ist die eigentliche Ursache für die relativ hohen Verluste an Ethylenglykol in der Prozesskolonne von PET-Anlagen.

Aus der internationalen Patentanmeldung WO 96/35654 ist ein Verfahren bekannt, bei dem das Prozessabwasser aus dem PET-Herstellungsprozess nach der Prozesskolonne (Kopfprodukt) teilweise kondensiert und im Anschluss an eine Inertgasstrippung einer Umkehrosmose unterworfen wird. Dabei erfolgt eine Aufkonzentrierung des im teilgereinigten Prozessabwasser verbliebenen Ethylenglykols durch das Anlegen eines Drucks, der größer ist als der osmotische Druck der aufkonzentrierten Lösung. Das Wasser wird also entgegen dem Bestreben zum osmotischen Konzentrationsausgleich durch die Membran gedrückt. Das Permeat besteht aus reinem Wasser, welches direkt ausgeleitet werden kann, so dass keine (aufwändige) Kläranlage mehr erforderlich ist. Zusätzlich erlaubt das Verfahren höhere Ethylenglykolkonzentrationen im Kopfprodukt der Prozesskolonne, was einerseits die benötigte Wärmeenergie für die Kolonne, und in Folge den Kühlmittelbedarf für die anschließende Kondensation, verringert. Darüber hinaus kann die Anzahl der benötigten Kolonnenböden reduziert werden. Das Retentat (Konzentrat) aus der Umkehrosmose wird dem Prozess wieder zugeführt, wobei eine Rückführung entweder in den Reaktor oder direkt in die Prozesskolonne vorgesehen ist. Durch dieses Verfahren wird die Rückgewinnung von Ethylenglykol mittels Umkehrosmose zwar gegenüber herkömmlichen Verfahren verbessert, das Problem des Verlusts an Ethylenglykol in Form von MDO wird jedoch weder erkannt noch gelöst.

Die Aufgabe der vorliegenden Erfindung besteht nun darin, ein Verfahren und eine Anlage bzw. Vorrichtung zur Verfügung zu stellen, das/die zur Rückgewinnung von Ethylenglykol bei der Herstellung von PET geeignet ist. Die Erfindung soll einerseits eine Einsparung an Investitions- und/oder Betriebskosten ermöglichen und andererseits umweltschonend umsetzbar sein. Eine einfache, funktionsgerechte Bauweise, ein geringer Anlagenaufwand und/oder eine Energieersparnis sind ebenfalls Ziele, deren Erreichung durch die vorliegende Erfindung angestrebt wird. Insbesondere soll durch eine Optimierung der Ethylenglykolrückgewinnung eine Reduktion des Rohmaterialverbrauchs bei minimalem Aufwand gewährleistet werden. Weitere Aufgaben und Vorteile der vorliegenden Erfindung ergeben sich aus den nachfolgenden Ausführungen.

Erfindungsgemäß wird die Aufgabe durch ein Verfahren zur Rückgewinnung von Ethylenglykol beim PET-Herstellungsprozess gelöst, wobei der PET-Herstellungsprozess eine Veresterungsreaktion beinhaltet, bei der Prozessabwasser entsteht, wobei das Prozessabwasser mindestens einem Trennschritt vorzugsweise einer Rektifikation unterzogen wird, bei welchem mindestens ein Teil der im Prozessabwasser vorhandenen Verunreinigungen abgetrennt wird und so ein teilgereinigtes Prozessabwasser entsteht, wobei zumindest ein Teil des teilgereinigten Prozessabwassers mit einem 2-Methyl-1,3-dioxolan-(MDO)-enthaltenden Fluid vorzugsweise einem oder mehreren Spalt-Ethylenglykol-(SEG)-Prozessströmen, in einem der Rektifikationskolonne vorgelagerten Behälter gemischt wird und in der entstehenden Mischung das vorhandene MDO ganz oder teilweise in Ethylenglykol und Acetaldehyd gespalten wird.

Im PET-Herstellungsprozess entsteht bei der Veresterungs- bzw. Kondensationsreaktion der Terephthalsäure mit Ethylenglykol (Prozess-)Wasser, welches die Veresterungs- und Kondensationsreaktoren zusammen mit anderen Stoffen als Brüden verlässt. Bei den genannten Stoffen (im folgenden gesamthaft als Verunreinigungen bezeichnet), handelt es sich u. A. um Ethylenglykol, Essigsäure und Acetaldehyd. Diese müssen entweder aus Gründen der Wirtschaftlichkeit zurückgewonnen oder zur Erfüllung von Umweltauflagen entfernt werden. Das Prozessabwasser, also die Summe aus (Prozess-)Wasser und Verunreinigungen, wird deshalb einem oder mehreren Trennschritten unterworfen, wobei eine erste Trennung meist auf destillativem Wege in einer Rektifikationskolonne stattfindet. Dort wird ein Großteil des vorhandenen Ethylenglykols abgetrennt, und das Kopfprodukt der Kolonne wird anschließend einem Kondensator zugeführt, wobei ein Teil des Acetaldehyds und anderer Leichtsieder in der Gasphase verbleiben und als Abluft einer thermischen oder katalytischen Verbrennung zugeleitet werden. Durch einen solchen ersten Trennschritt entsteht ein teilgereinigtes Prozessabwasser, welches nach der bisherigen Praxis oft noch über einen Stripper geführt wird, bevor es in eine Kläranlage geleitet oder direkt als Abwasser ausgeschleust wird.

Neben teilgereinigtem Prozessabwasser fallen im PET-Herstellungsprozess erhebliche Mengen an verunreinigtem Ethylenglykol (Spalt-Ethylenglykol, SEG) an, welche unter anderem MDO enthalten. Das SEG stammt insbesondere vom Vakuumsystem und dem Ethylenglykolkreislauf der Vorpolykondensation. Die SEG-Prozessströme aus den verschiedenen Prozessschritten des PET-Herstellungsverfahrens werden oft in einem Sammelbehälter (SEG-Behälter bzw. SEG-Sammelbehälter) zusammengeführt, anschließend einem Trennschritt in einer Rektifikationskolonne unterworfen und dann dem PET-Herstellungsprozess wieder zugeleitet.

Beim MDO handelt es sich um einen Leichtsieder, der in der Rektifikationskolonne nicht zurückgehalten werden kann, d.h. über Kopf die Kolonne zusammen mit dem (Prozess-)Wasser verlässt. Der Großteil des MDO wird danach gemeinsam mit dem (Prozess-)Wasser kondensiert und als Teil des teilgereinigten Prozessabwassers ausgeschleust. Ein geringer Teil des MDO wird über die Abluft abgegeben. Die Existenz des MDO ist die eigentliche Ursache für die relativ hohen Verluste an EG in der Kolonne von PET-Anlagen. Unter neutralen und leicht alkalischen pH-Bedingungen ist MDO stabil, im sauren Bereich zerfällt MDO in Abhängigkeit vom Wassergehalt in die Ausgangsstoffe Ethylenglykol und Acetaldehyd. Die Bildung als auch Spaltung des MDO sind säurekatalysiert.

Es wurde erkannt, dass das teilgereinigte Prozessabwasser (oder ein Teil dessen) dazu verwendet werden kann, den Wassergehalt in mit MDO verunreinigtem Ethylenglykol (SEG) aus dem PET-Herstellungsprozess soweit zu erhöhen, dass durch Verschiebung des chemischen Gleichgewichtes eine Spaltung eines wesentlichen Teils des vorhandenen MDO in Ethylenglykol und Acetaldehyd erfolgt. Durch Zufuhr von sauren Komponenten im teilgereinigten Prozessabwasser wird eine Beschleunigung der MDO-Spaltung ermöglicht. Trotzdem ist eine gewisse Verweilzeit für den Ablauf der MDO-Spaltungsreaktion erforderlich. Aus diesem Grund werden das teilgereinigte Prozessabwasser und das Spaltglykol in einem Behälter zusammengeführt, aus dem dann die Rektifikationskolonne gespeist wird. Der Behälter liefert die für die Spaltungsreaktion notwendige Verweilzeit. Gegenüber einem Rektifikationsprozess, der keine vorgelagerte MDO-Spaltung vorsieht, entsteht dadurch der Vorteil, dass das Rückflussverhältnis in der Prozesskolonne aufgrund des geringeren MDO-Gehalts der Brüden verringert werden kann, was aus wirtschaftlichen Gründen vorteilhaft ist. Als weiteres Reaktionsprodukt aus der MDO-Spaltung wird Acetaldehyd gebildet. Dabei handelt es sich um einen Leichtsieder, der via Prozesskolonne teilweise über die Gasphase einer Abluftbehandlung zugeführt werden kann.

Unter einem teilgereinigten Prozessabwasser soll in der vorliegenden Anmeldung Prozessabwasser verstanden werden, welches mindestens einen Trennschritt durchlaufen hat. Als bevorzugter erster Trennschritt ist eine destillative Aufarbeitung in einer Kolonne vorgesehen, wobei das entstehende teilgereinigte Prozessabwasser vorzugsweise die in Tabelle 1 aufgeführten Stoffe in den angegebenen Mengen beinhaltet. Wie aus der Tabelle hervorgeht, weist auch das teilgereinigte Prozessabwasser einen signifikanten MDO-Gehalt auf.

Alle hier genannten Prozentangaben sind - wenn nichts anderes bestimmt ist - als Gewichtsprozente aufzufassen. Die Basis ist der jeweils genannte Massenstrom.

Unter dem Begriff "*Prozess*" bzw. "*PET-Herstellungsprozess*" *oder* "*PET-Herstellungsverfahren*" ist der Gesamtprozess zur Herstellung von PET zu verstehen, inklusive aller Stoffumwandlungen und Stoffströme. Entsprechend ist mit "*Anlage*" die Gesamtanlage zur Herstellung von PET gemeint.

Angaben wie "*wasser mit*" oder "*Ethylenglykol mit*" bezeichnen die Hauptkomponente des jeweiligen Stoffstroms, also die Restmenge nach Abzug der Verunreinigungen. Bevorzugt weisen diese einen Gehalt der Hauptkomponente von mindestens 30 Gew.-% auf, wobei mindestens 60 Gew.-% und insbesondere mindestens 90 Gew.-% besonders bevorzugt sind.

Die Begriffe "*Wasser*", "*(Prozess-)Wasser*" bzw. "*Prozessabwasser*" machen keine Aussage zum Aggregatzustand. Das teilgereinigte Prozessabwasser kann folglich nicht nur flüssig, sondern auch gasförmig vorliegen.

**Tabelle 1**

| *Teilgereinigtes Prozessabwasser nach Prozesskolonne und Kühler:* | | |
|---|---|---|
| Massenstrom: | 185 - 195 kg/t PET | |
| Wasser mit | | |
| Ethylenglykol: | 0,4 - 0,6 % | |
| 2-Methyl-1,3-dioxolan: | 0,3 - 0,6 % | |
| Essigsäure: | 0,1 - 0,2 % | *) |
| | 0,01 - 0,02 % | **) |
| 1,4-Dioxan: | 0,04 - 0,06 % | |
| Acetaldehyd: | 0,9 - 1,5 % | |
| Temperatur: | 30 - 40 °C | |
| *) Bei Einsatz von Antimonacetat als Katalysator im PET-Herstellungsprozess | | |
| **) Bei Einsatz von Antimontrioxid als Katalysator im PET-Herstellungsprozess | | |

Es kann vorteilhaft sein, das teilgereinigte Prozessabwasser vor dem Mischen mit SEG noch einem oder mehreren weiteren Trennschritten zu unterwerfen, wobei es sich dabei bevorzugt um eine (weitere) Prozesskolonne und/oder einen Stripper handelt. Gestripptes, teilgereinigtes Prozessabwasser enthält vorzugsweise die in Tabelle 2 aufgeführten Stoffe in den angegebenen Mengen.

**Tabelle 2**

| *Teilgereinigtes Prozessabwasser nach Prozesskolonne und Stripper:* | | |
|---|---|---|
| Massenstrom: | 170 - 180 kg/t PET | |
| Wasser mit | | |
| Ethylenglykol: | 0,2 - 0,6 % | |
| 2-Methyl-1,3-dioxolan: | 0,01 - 0,05 % | |
| Essigsäure: | 0,06 - 0,2 % | *) |
| | 0,006 - 0,02 % | **) |
| 1,4-Dioxan: | 0,01 - 0,05 % | |
| Acetaldehyd: | 0,01 - 0,03 % | |
| Temperatur: | 30 - 60 °C | |
| *) Bei Einsatz von Antimonacetat als Katalysator im PET-Herstellungsprozess | | |
| **) Bei Einsatz von Antimontrioxid als Katalysator im PET-Herstellungsprozess | | |

Das Strippen findet dabei bevorzugt mit Luft als Stripgas statt.

Besonders vorteilhaft ist das oben genannte erfindungsgemäße Verfahren dann, wenn mindestens ein Teil des teilgereinigten Prozessabwassers vor der Mischung mit dem MDO-enthaltenden Fluid mindestens einer Umkehrosmose und/oder Ultra/Nanofiltration unterzogen wird, wobei vorzugsweise derjenige Teil des Prozessabwassers, der bei der Umkehrosmose und/oder Ultra/Nanofiltration als Retentat anfällt, zumindest teilweise zur Mischung mit dem Methyldioxolan-enthaltenden Fluid eingesetzt wird.

Aus einer solchen Verfahrensführung ergeben sich verschiedene Vorteile. Der Einsatz einer Umkehrosmose ermöglicht je nach Ausführung (ein- bis dreistufig), auf eine (aufwändige) Abwasserreinigung zu verzichten, da das Permeat aus schwach bis nicht belastetem Wasser besteht. Erfindungsgemäß kann jedoch auch bei Einsatz einer Umkehrosmose und/oder Ultra/Nanofiltration in Abhängigkeit von der Ausführung der Membranverfahren und den gültigen Umweltvorschriften dennoch eine finale Abwasserreinigung vorgesehen sein, wobei diese jedoch aufgrund der Vorreinigung des Prozessabwassers (hier: Permeat) einfacher ausgestaltet sein und mit weniger Aufwand betrieben werden kann. Zusätzlich führt die Verwendung einer Umkehrosmose zu geringeren MDO-Konzentrationen im Kopfprodukt der Rektifikationskolonne, was die benötigte Wärmeenergie für die Kolonne (Rückfluss) und in Folge den Kühlmittelbedarf für die anschließende Kondensation verringert. Darüber hinaus kann die Anzahl der benötigten Kolonnenböden reduziert werden.

Neben den genannten Vorteilen wird durch das erfindungsgemäße Verfahren, d.h. durch die Spaltung des MDO, zusätzlich Ethylenglykol zurückgewonnen. Dabei wird vorzugsweise das Retentat (Konzentrat) aus der Umkehrosmose, d.h. der an Ethylenglykol angereicherte Teil des teilgereinigten Prozessabwassers, zur Erhöhung des Wassergehalts im SEG verwendet. Zwar muss das in den PET-Herstellungsprozess, vorzugsweise in die Kolonne, zurückgeführte (Prozess-)Wasser verdampft und anschließend kondensiert werden. Im Gegensatz zum Verfahren nach dem Stand der Technik wird dieser energetische Nachteil durch das zurückgewonnene Ethylenglykol aber mehr als kompensiert. Entgegen dem Stand der Technik muss daher keine Minimierung des Retentatvolumens mittels Umkehrosmose angestrebt werden. Das erfindungsgemäße Verfahren erlaubt deshalb, den Druck - und damit den Energieaufwand - während der Umkehrosmose geringer zu halten. Darüber hinaus wird ein Teil des zugeführten (Prozess-)Wassers bei der Spaltung des MDO verbraucht. Die Umkehrosmose und/oder Ultra/Nanofiltration wird vorzugsweise bei Temperaturen von 20 - 50°C und/oder Drücken von 5 - 70 bar(g) durchgeführt.

Teilgereinigtes, insbesondere nicht gestripptes, Prozessabwasser enthält signifikante Mengen an 1,4-Dioxan (s. Tab. 1 und 2). Da 1,4-Dioxan biologisch praktisch nicht abbaubar ist, werden die häufig geforderten niedrigen Ablaufwerte nach Kläranlage bezüglich chemischer Summenparameter wie z.B. CSB (chemischer Sauerstoffbedarf) und TOC (organischer Gesamtkohlenstoff) nicht erreicht. Mit der geschilderten Anwendung der Umkehrosmose und/oder Ultra/Nanofiltration wird ein Großteil des im Prozessabwassers enthaltenem 1,4-Dioxans in den PET-Herstellungsprozess zurückgeführt und von dort über das Abgas in einer thermischen oder katalytischen Abluftreinigung problemlos behandelt. Das von 1,4-Dioxan abgereicherte Permeat wird einer Kläranlage zugeführt, wobei nun mit der identischen Ausrüstung der Kläranlage eine deutlich verbesserte Ablaufqualität bezüglich chemischer Summenparameter wie z.B. CSB und TOC erreicht werden kann.

Nach Durchlaufen der Umkehrosmose und/oder Ultra/Nanofiltration beinhaltet das teilgereinigte Prozessabwasser vorzugsweise die in Tabelle 3 (Retentat) und Tabelle 4 (Permeat) aufgeführten Stoffe in den angegebenen Mengen.

**Tabelle 3**

| *Teilgereinigtes Prozessabwasser* / *Retentat aus Umkehrosmose:* | | | |
|---|---|---|---|
| Verfahren mit Stripper: | | | |
| | Massenstrom: | 4 - 22 kg/t PET | |
| | Wasser mit | | |
| | Ethylenglykol: | 3 - 10 % | |
| | 2-Methyl-1,3-dioxolan: | 0,1 - 0,6 % | |
| | Essigsäure: | 0,5 - 2 % 0,05 - 0,2 % | *) **) |
| | 1,4-Dioxan: | 0,1-0,6% | |
| | Acetaldehyd: | nicht relevant | |
| | Temperatur: | 20 - 40 °C | |
| | | | |

| Verfahren ohne Stripper: | | | |
|---|---|---|---|
| | Massenstrom: | 4 - 22 kg/t PET | |
| | Wasser mit | | |
| | Ethylenglykol: | 3 - 10 % | |
| | 2-Methyl-1,3-dioxolan: | 3 - 10 % | |
| | Essigsäure: | 0,7 - 2 % | *) |
| | | 0,07 - 0,2 % | **) |
| | 1,4-Dioxan: | 0,5 - 0,8 % | |
| | Acetaldehyd: | nicht relevant | |
| | Temperatur: | 20 - 40 °C | |
| *) Bei Einsatz von Antimonacetat als Katalysator im PET-Herstellungsprozess | | | |
| **) Bei Einsatz von Antimontrioxid als Katalysator im PET-Herstellungsprozess | | | |

**Tabelle 4**

| *Teilgereinigtes Prozessabwasser* / *Permeat aus Umkehrosmose:* | | | |
|---|---|---|---|
| Verfahren mit Stripper: | | | |
| | Massenstrom: | 152 - 170 kg/t PET | |
| | Wasser mit | | |
| | Ethylenglykol: | 0,05 - 0,15 % | |
| | 2-Methyl-1,3-dioxolan: | ca. 0,005 % | |
| | Essigsäure: | 0,05 - 0,1 % | *) |
| | | 0,005 - 0,01 % | **) |
| | 1,4-Dioxan: | 0,002 - 0,006 % | |
| | Acetaldehyd: | nicht relevant | |
| | Temperatur: | 20 - 40 °C | |
| | | | |

| Verfahren ohne Stripper: | | | |
|---|---|---|---|
| | Massenstrom: | 168 - 185,7 kg/t PET | |
| | Wasser mit | | |
| | Ethylenglykol: | 0,05 - 0,15 % | |
| | 2-Methyl-1,3-dioxolan: | ca. 0,07 % | |
| | Essigsäure: | 0,07 - 0,12 % 0,007 - 0,012 % | *) **) |
| | 1,4-Dioxan: | 0,002 - 0,006 % | |
| | Acetaldehyd: | nicht relevant | |
| | Temperatur: | 20 - 40 °C | |
| | | | |
| Ableitung als Abwasser zur Endreinigung in einer chemisch-biologischen Abwasserreinigung. | | | |
| *) Bei Einsatz von Antimonacetat als Katalysator im PET-Herstellungsprozess | | | |
| **) Bei Einsatz von Antimontrioxid als Katalysator im PET-Herstellungsprozess | | | |

Bei Einsatz einer Umkehrosmose ist mit den in der folgenden Tabelle 5 aufgeführten Rückhaltegraden zu rechnen:

**Tabelle 5**

| *Rückhaltegrade* der Umkehrosmose gegenüber:* | |
|---|---|
| Ethylenglykol: | 75 - 85 % |
| 2-Methyl-1,3-dioxolan: | 80 - 90 % |
| Essigsäure: | 45 - 55 % |
| 1,4-Dioxan: | 85 - 95 % |
| Acetaldehyd: | nicht relevant |
| *) Verfahren mit/ohne Stripper | |

Unter SEG-Prozessströmen sind in der vorliegenden Anmeldung alle mit MDO verunreinigten Prozessströme im PET-Herstellungsverfahren zu verstehen, welche im wesentlichen Ethylenglykol beinhalten. Das im erfindungsgemäßen Verfahren verwendete SEG bzw. die SEG-Prozessströme weisen vorzugsweise einen Ethylenglykol-Gehalt von mindestens 30 Gew.-% auf, wobei ein Gehalt von mindestens 60 Gew.-% und insbesondere mindestens 90 Gew.-% besonders bevorzugt ist. Bei den erfindungsgemäß eingesetzten SEG-Prozessströmen handelt es sich bevorzugt um die Stoffströme, aus denen der Ethylenglykol-Kreislauf der Vorpolykondensation besteht, sowie um SEG-Prozessströme aus dem Vakuumsystem der Vorpolykondensation.

Das SEG aus der Vorpolykondensation beinhaltet vorzugsweise die in Tabelle 6 aufgeführten Stoffe in den angegebenen Mengen.

**Tabelle 6**

| *SEG-Strom*** von Vorpolykondensation in SEG-Sammelbehälter:* | | |
|---|---|---|
| Massenstrom: | 90 - 110 kg/t PET | |
| Ethylenglykol mit | | |
| Wasser: | 3 - 7 % | |
| 2-Methyl-1,3-dioxolan: | 0,2 - 0,7 % | |
| Essigsäure: | ca. 0,1 % | *) |
| | ca. 0,01 % | **) |
| 1,4-Dioxan: | 0,02 - 0,04 % | |
| Acetaldehyd: | nicht relevant | |
| Temperatur: | 35 - 60 °C | |
| *) Bei Einsatz von Antimonacetat als Katalysator im PET-Herstellungsprozess | | |
| **) Bei Einsatz von Antimontrioxid als Katalysator im PET-Herstellungsprozess | | |
| ***) Verfahren mit/ohne Stripper | | |

Da das SEG bzw. die SEG-Prozessströme meist in flüssiger Form vorliegen und das Prozessabwasser zur Behandlung in der Umkehrosmoseeinheit ebenfalls flüssig zur Verfügung stehen muss, ist es bevorzugt, SEG und teilgereinigtes Prozessabwasser in flüssiger Form zu mischen. Jedoch ist auch eine Mischung denkbar, in der sowohl das SEG als auch das teilgereinigte Prozessabwasser in Gasform vorliegen, oder eine Mischung, bei der eine der zu mischenden Komponenten ganz oder teilweise gasförmig zur Verfügung steht, während die andere ganz oder teilweise flüssig ist.

Gemäß der vorliegenden Erfindung ist vor oder während der Mischung des SEG mit dem teilgereinigten Prozessabwasser bevorzugt eine Zusammenführung von zwei oder mehreren der SEG-Prozessströme des PET-Herstellungsprozesses vorgesehen. Bei der Zusammenführung der SEG-Prozessströme kommt vorzugsweise ein SEG-Sammelbehälter zum Einsatz, wie er in den meisten Anlagen nach dem Stand der Technik bereits vorhanden ist. Zusätzlich können ein oder mehrere weitere Behälter vorgesehen sein, in dem/denen die Mischung des SEG mit dem teilgereinigten Prozessabwasser und die Umsetzung des MDO stattfinden. Jedoch ist es erfindungsgemäß bevorzugt, dass die Mischung ebenfalls im SEG-Sammelbehälter erfolgt, welcher dazu u. A. um eine oder mehrere Zuleitungen für teilgereinigtes Prozessabwasser ergänzt wird. Auf diese Weise wird kein separater Behälter für die Umsetzung des MDO benötigt, was die Anzahl der erforderlichen Anlagenteile reduziert, womit eine Reduktion des Investitions- und Betriebsaufwands erreicht wird. Es können außerdem Mischelemente vorgesehen sein, welche sich beispielsweise entweder im Behälter selbst befinden oder als statische Mischelemente vor dem Behälter angeordnet sind. Entsprechend kann der Behälter statt separater Zuleitungen für SEG und teilgereinigtes Prozessabwasser auch eine oder mehrere Zuleitungen aufweisen, welche SEG und teilgereinigtes Prozessabwasser gemeinsam zuführen. Der (ein oder mehrere) Behälter ist vorzugsweise so gestaltet, dass mindestens 20 Gew.-%, vorzugsweise mindestens 40 Gew.-%, des im zugeführten SEG und/oder im zugeführten teilgereinigten Prozesswasser vorhandenen MDO in Ethylenglykol und Acetaldehyd spaltbar sind. Entsprechend ist ein genanntes Verfahren zur Rückgewinnung von Ethylenglykol besonders bevorzugt, wenn im (ein oder mehrere) Behälter 20-80 Gew.-%, vorzugsweise 30-70 Gew.-%, besonders bevorzugt 40-60 Gew.-% des im zugeführten SEG und/oder im zugeführten teilgereinigten Prozesswasser vorhandenen MDO in Ethylenglykol und Acetaldehyd gespalten werden, bzw. die MDO-Spaltungsrate im Behälter diese Werte erreicht.

Neben dem oben genannten Verfahren umfasst die vorliegende Erfindung auch eine Anlage zur Herstellung von PET mit einem oder mehreren Veresterungs- und/oder Umesterungsreaktoren sowie einer oder mehrerer Einrichtungen zur Herstellung eines teilgereinigten Prozessabwassers durch Abtrennung von im Prozessabwasser vorhandenen Verunreinigungen, bei der es sich vorzugsweise um eine Rektifikationskolonne handelt, wobei die Anlage einen oder mehrere Behälter aufweist, welche zumindest eine Zuleitung für teilgereinigtes Prozessabwasser sowie mindestens eine Zuleitung für ein MDO-enthaltendes Fluid (vorzugsweise ein Ethylenglykol-Prozessstrom) und/oder eine oder mehrere Zuleitungen für eine Mischung aus teilgereinigtem Prozessabwasser und einem MDO-enthaltenden Fluid aufweisen.

Eine genannte Anlage ist als besonders vorteilhaft zu betrachten, wenn sie eine Umkehrosmoseeinrichtung und/oder Ultra/Nanofiltrationseinrichtung aufweist, welche verfahrenstechnisch vor dem Behälter angeordnet ist.

Die Anlage weist in diesem Falle vorzugsweise eine oder mehrere Leitungen auf, welche denjenigen Teil des teilgereinigten Prozessabwassers, welcher als Retentat in der Umkehrosmoseeinheit und/oder Ultra/Nanofiltrationseinheit anfällt (oder einen Teil davon), direkt oder indirekt dem Behälter zuführt. Die Anlage weist also vorzugsweise eine oder mehrere Leitungen auf, die zwischen der Umkehrosmoseeinheit und/oder der Ultra/Nanofiltrationseinheit und dem Behälter verlaufen.

Der Zufluss an teilgereinigtem Prozessabwasser in den Behälter wird vorzugsweise in Abhängigkeit des Wassergehalts der Mischung im Behälter, welcher bevorzugt periodisch gemessen wird, gesteuert. Ist eine Umkehrosmoseeinheit und/oder eine Ultra/Nanofiltrationseinheit vorgesehen, so kann auch der Druck in der Umkehrosmoseeinheit und/oder Ultra/Nanofiltrationseinheit, bzw. der Grad der Aufkonzentrierung, in Abhängigkeit des Wasseranteils im Behälter gesteuert werden, um eine erhöhte oder geringere Anreicherung an Ethylenglykol und/oder Essigsäure bzw. einen höheren oder niedrigeren Wassergehalt zu erreichen. Es kann auch vorteilhaft sein, die Trennschärfe der Prozesskolonne in Abhängigkeit des Wassergehalts im Behälter zu steuern, beispielsweise über das Einstellen des Rückflussverhältnisses.

Eine Anlage, wie sie oben beschrieben wird, ist dann besonders vorteilhaft, wenn das Volumen des Behälters so bemessen ist, dass die Verweilzeit 0,2 - 4 Stunden, vorzugsweise 0,5 - 3 Stunden, besonders bevorzugt 1 - 2 Stunden beträgt.

Auf diese Weise wird ein Abbau eines substanziellen Teils des MDO gewährleistet. Der Behälter dient gleichzeitig als Puffer für SEG und/oder teilgereinigtes Prozessabwasser. Unter "Verweilzeit" ist die Verweilzeit des Behälterinhalts, d.h. der Mischung aus teilgereinigtem Prozessabwasser und SEG, zu verstehen.

Nach einer weiteren bevorzugten Ausgestaltungsform erfolgt im oben genannten Verfahren bzw. in der genannten Anlage die Spaltung des MDO in Anwesenheit von einem oder mehreren Katalysatoren, wobei es sich bei den Katalysatoren vorzugsweise um einen oder mehrere der folgenden handelt: "organische aliphatische und aromatische Säuren, vorzugsweise Essigsäure".

Ein genanntes Verfahren ist insbesondere auch dann vorteilhaft, wenn eine oder mehrere im Prozessabwasser enthaltene Säuren, vorzugsweise Essigsäure, (bevorzugt mittels Umkehrosmose) aufkonzentriert und als Katalysatoren für die Spaltung des MDO eingesetzt werden.

Dies ist ein weiterer Grund, weswegen beim erfindungsgemäßen Verfahren der Einsatz einer Umkehrosmose und/oder Ultra/Nanofiltration favorisiert wird. Durch Umkehrosmose und/oder Ultra/Nanofiltration kann nicht nur eine Aufkonzentrierung von MDO im Prozessabwasser und eine Rückgewinnung von Ethylenglykol erreicht werden, sondern auch eine Aufkonzentrierung anderer Verunreinigungen bzw. Inhaltsstoffe, insbesondere organischer Säuren wie Essigsäure. Diese werden somit im Prozess gehalten und können beispielsweise als Katalysatoren bei der MDO-Spaltung dienen. Ist die Menge an Katalysatoren zu gering, ist zusätzlich auch eine Zugabe von außen denkbar. Vorzugsweise handelt es sich bei den Katalysatoren um solche Stoffe, die die anderen mit der PET-Herstellung im Zusammenhang stehenden Prozesse im wesentlichen nicht negativ beeinflussen und so im Kreislauf geführt werden können. Das bedeutet, dass die Katalysatoren, bei welchen es sich vorzugsweise um saure Verbindungen handelt, über das teilgereinigte Prozessabwasser und/oder die SEG-Prozessströme dem Behälter wieder zugeführt werden können und damit die MDO-Spaltung beschleunigen können.

Bei der Spaltung des MDO handelt es sich um eine Gleichgewichtsreaktion. Folglich wird sich bei Zunahme der Wasserkonzentration in der Mischung das chemische Gleichgewicht in Richtung der MDO-Spaltprodukte Ethylenglykol und Acetaldehyd verschieben. Da das Wasser in der Prozesskolonne wieder verdampft werden muss, ist es vorteilhaft, den Wasseranteil in der Mischung gering zu halten. Die Umsetzung des MDO im Behälter erfolgt deshalb bevorzugt unter Bedingungen, wie sie in Tabelle 7 aufgeführt sind. So wird einerseits eine Spaltung eines wesentlichen Teils des MDO gewährleistet und andererseits wird damit dem zusätzlichen Aufwand im Zusammenhang mit einer Rückführung des Wassers in den Prozess Rechnung getragen.

**Tabelle 7**

| Bedingungen für MDO-Spaltung im SEG-Sammelbehälter* | |
|---|---|
| | - Verweilzeit: 0,5 - 3 h, bevorzugt: 1 - 2 h |
| | - Temperatur: 35 - 95 °C, bevorzugt: 40 - 60 °C |
| | - Konzentration an Wasser: ≥ 10 %, bevorzugt: 15 - 25 % |
| | - Konzentration an Säure: 0,1 - 1 %, bevorzugt: 0,2 - 0,4 % |
| | - Abluftableitung: Absaugung des gebildeten Acetaldehyds, Abführung |
| | über Abluftsammelleitung zur thermischen oder katalytischen Abluftbehandlung |
| | - Spaltungsrate an MDO: 20 - 80 %, bevorzugt: 40 - 60 % |
| *) Verfahren mit/ohne Stripper | |

Wie auch in Tabelle 7 aufgeführt, besteht eine weitere Möglichkeit, das Gleichgewicht in Richtung des MDO zu verschieben, darin, Acetaldehyd aus dem Behälter abzusaugen. Der Druck im Behälter wird also vorzugsweise so eingestellt und gegebenenfalls auf die übrigen Bedingungen (vgl. Tabelle 7) abgestimmt, dass die in Tabelle 7 genannte Spaltungsrate erreicht wird.

Die Menge an zurückgewonnenem Ethylenglykol ist in Tabelle 8 dargestellt:

**Tabelle 8**

| *Ethylenglykol-Rückgewinnungsrate:* | | |
|---|---|---|
| Bei Verwendung einer Prozesskolonne und eines Strippers: | | |
| | Generelle Rückgewinnungsraten an Ethylenglykol: | |
| | - durch Umkehrosmose (UO): | 0,3 - 0,9 kg/t PET |
| | - durch MDO-Spaltung: | 0,1 - 0,5 kg/t PET |
| | | |
| | Für gestripptes teilgereinigtes | Prozessabwasser gelten folgende |
| | Ethylenglykol-Rückgewinnungsraten: | |
| | - UO + MDO-Spaltung: | 0,5 - 1,3 kg/t PET |
| | Durch Anpassung der Betriebsparameter des vorhandenen Strippersystems an die Umkehrosmose/MDO-Spaltung kann die EG-Rückgewinnungsrate auf mindestens 1,5 kg/t erhöht werden. | |
| | | |
| Bei Verwendung einer Prozesskolonne, ohne Einsatz eines Strippers: | | |
| | Generelle Rückgewinnungraten an Ethylenglykol: | |
| | - durch UO: | 0,6 - 1,0 kg/t PET |
| | - durch MDO-Spaltung: | 0,1 - 1,0 kg/t PET |
| | | |
| | Für ungestripptes teilgereinigtes Prozessabwasser gelten folgende Ethylengly kol-Rückgewinnungsraten: | |
| | - UO + MDO-Spaltung: | 0,8 - 1,8 kg/t PET |

Von besonderer Bedeutung ist ein oben genanntes Verfahren dann, wenn die Mischung aus SEG und teilgereinigtem Prozessabwasser einen Wassergehalt von mindestens 10 Gew.-%, vorzugsweise 13 bis 45 Gew.-%, besonders bevorzugt 15 - 25 Gew.-% aufweist. Vorzugsweise wird der Zustrom an SEG und teilgereinigtem Prozessabwasser zum Behälter so gesteuert, dass oben genannter Wassergehalt der Mischung erreicht wird.

Ist eine MDO Spaltungsrate von etwa 50% vorgesehen, so beinhaltet die Mischung aus SEG und teilgereinigtem Prozessabwasser vorzugsweise die in Tabelle 9 aufgeführten Stoffe in den angegebenen Mengen. Dabei handelt es sich bevorzugt um denjenigen Prozessstrom, welcher zurück in den PET-Herstellungsprozess geführt wird.

**Tabelle 9**

| *Mischung: SEG-Strom* + *teilgereinigtes Prozessabwasser (Retentat) nach MDO-Spaltung zu 50 %* | | |
|---|---|---|
| Verfahren mit Stripper: | | |
| Massenstrom: | 94 - 132 kg/t PET | |
| Ethylenglykol mit | | |
| Wasser: | 10 - 20 % | |
| 2-Methyl-1,3-dioxolan: | 0,2 - 0,7 % | |
| Essigsäure: | 0,1 - 0,3 % 0,01 - 0,03 % | *) **) |
| 1,4-Dioxan: | 0,05 - 0,08 % | |
| Acetaldehyd: | nicht relevant | |
| Temperatur: | 35 - 60 °C | |
| | | |
| Verfahren ohne Stripper: | | |
| Massenstrom: | 94 - 132 kg/t PET | |
| Ethylenglykol mit | | |
| Wasser: | 10-20% | |
| 2-Methyl-1,3-dioxolan: | 0,3 - 0,8 % | |
| Essigsäure: | 0,2 - 0,4 % | *) |
| | 0,02 - 0,04 % | **) |
| 1,4-Dioxan: | 0,08 - 0,12 % | |
| Acetaldehyd: | nicht relevant | |
| Temperatur: | 35 - 60 °C | |
| *) Bei Einsatz von Antimonacetat als Katalysator im PET-Herstellungsprozess | | |
| **) Bei Einsatz von Antimontrioxid als Katalysator im PET-Herstellungsprozess | | |

Wie erwähnt, erfolgt die Rückführung des teilgereinigten Prozessabwassers in den PET-Herstellungsprozess in Form einer Mischung mit dem SEG. Diesbezüglich ist ein oben bezeichnetes Verfahren besonders vorteilhaft, wenn die Mischung (ganz oder teilweise) nach der Spaltung zumindest eines Teils des MDO dem PET-Herstellungsprozess an einer oder mehreren Stellen zugeführt wird. Besonders bevorzugt ist dabei eine Zuführung zu einer Prozesskolonne der PET-Polykondensationsanlage und/oder einer Ethylenglykolrückgewinnungsanlage vorgesehen.

Die in der Anmeldung genannten Stoffströme stehen in einem Abhängigkeitsverhältnis zueinander und die Entscheidung, wie deren Zusammensetzung und sonstigen Eigenschaften zu wählen sind, hängt vom Ziel, das erreicht werden soll, ab. Es wurde festgestellt, dass Stoffströme, welche in Bezug auf ihre Zusammensetzung in den Konzentrationsbereichen liegen, wie sie in den oben aufgeführten Tabellen angegeben sind, bevorzugt sind. Diese gewährleisten eine MDO-Umsetzungsrate, welche in Bezug auf Ethylenglykol-Bildung und aus wirtschaftlichen Gründen besonders vorteilhaft ist. Die Erfindung beschränkt sich jedoch nicht auf die angegebenen Werte.

### Beschreibung der Zeichnung:

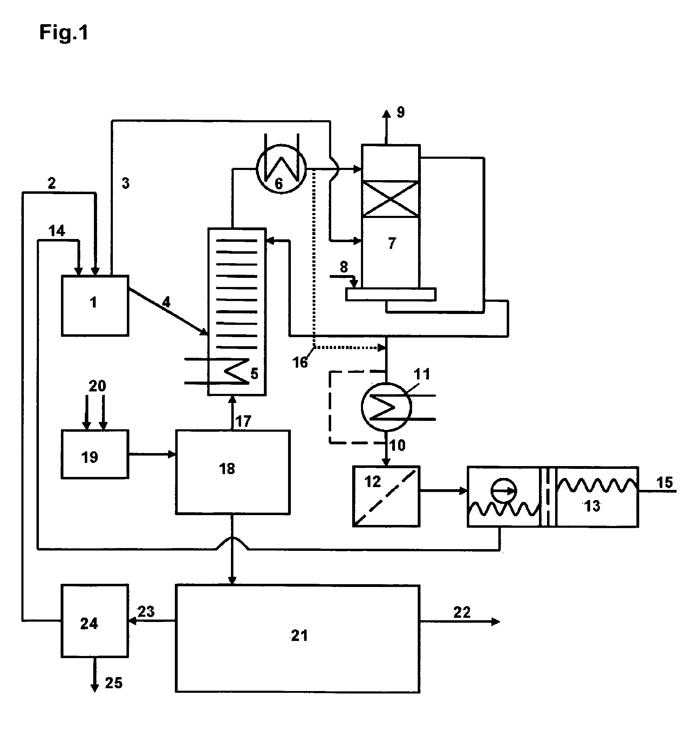

Die **Fig.1** stellt beispielhaft ein Verfahrensschema dar, welches die für die Ethylenglykol-Rückgewinnung im PET-Herstellungsprozess relevanten bzw. bevorzugten Verfahrensschritte, Vorrichtungsteile und Stoffströme abbildet.

Nachfolgend soll die Erfindung anhand von Beispielen erläutert werden, wobei sie sich natürlich nicht auf diese Ausführungsformen beschränkt.

Die Ausführungsbeispiele zeigen die Zusammensetzung der Stoffströme, wie sie in einer erfindungsgemäßen Anlage zur PET-Herstellung mit einer Kapazität von 330 t/d auftreten können. Als erster Trennschritt bzw. als Trenneinrichtung für das Prozessabwasser ist eine Prozesskolonne vorgesehen. Es findet außerdem eine Ethylenglykol-Anreicherung mittels Umkehrosmose statt.

### Abkürzungen und Hinweise:

- In Bezug auf die Bezeichnung der Stoffströme ("Strom [Nr.]") sei auf das in Fig. 1 dargestellte Verfahrensschema verwiesen.
- Die Angabe "*)" bedeutet, dass Antimonacetat als Katalysator im PET-Herstellungsprozess eingesetzt wurde, während "**)" auf einen Einsatz von Antimontrioxid als Katalysator im PET-Herstellungsprozess hindeutet.

### Beispiel 1:

Die folgenden Tabellen zeigen die Stoffströme in einem PET-Herstellungsprozess, wobei das Prozessabwasser nach Durchlaufen einer Prozesskolonne einem weiteren Trennschritt unterworfen wird (Strippung mit Luft):
**Strom 10** (Teilgereinigtes Prozessabwasser nach Stripper)

| | | |
|---|---|---|
| Massenstrom: | 2400 kg/h | |
| Wasser mit | | |
| Ethylenglykol: | 0,4 % | |
| 2-Methyl-1,3-dioxolan: | 0,03 % | |
| Essigsäure: | 0,15 % | *) |
| 1,4-Dioxan: | 0,03 % | |
| Acetaldehyd: | nicht relevant | |
| Temperatur: | 35 - 45 °C | |

**Strom 14** (Teilgereinigtes Prozessabwassers: Retentat aus Umkehrosmose)

| | | |
|---|---|---|
| Massenstrom: | 180 kg/h | |
| Wasser mit | | |
| Ethylenglykol: | 4,3 % | |
| 2-Methyl-1,3-dioxolan: | 0,35 % | |
| Essigsäure: | 1 % | *) |
| 1,4-Dioxan: | 0,35 % | |
| Acetaldehyd: | nicht relevant | |
| Temperatur: | 30 - 40 °C | |

**Rückhaltegrade der Umkehrosmose gegenüber:**

| | |
|---|---|
| Ethylenglykol: | 80 % |
| 2-Methyl-1,3-dioxolan: | 85 % |
| Essigsäure: | 50 % |
| 1,4-Dioxan: | 90 % |
| Acetaldehyd: | nicht relevant |

**Strom 15** (Teilgereinigtes Prozessabwasser: Permeat aus Umkehrosmose)

| | | |
|---|---|---|
| Massenstrom: | 2220 kg/h | |
| Wasser mit | | |
| Ethylenglykol: | 0,1 % | |
| 2-Methyl-1,3-dioxolan: | 0,005 % | |
| Essigsäure: | 0,08 % | *) |
| 1,4-Dioxan: | 0,003 % | |
| Acetaldehyd: | nicht relevant | |
| Temperatur: | 30 - 40 °C | |
| Ableitung als Abwasser zur Endreinigung in einer chemisch-biologischen Abwasserreinigung | | |

**Strom 2** (SEG von Vorpolykondensation)

| | | |
|---|---|---|
| Massenstrom: | 1375 kg/h | |
| Ethylenglykol mit | | |
| Wasser: | 5 % | |
| 2-Methyl-1,3-dioxolan: | 0,45 % | |
| Essigsäure: | 0,1 % | *) |
| 1,4-Dioxan: | 0,03 % | |
| Acetaldehyd: | nicht relevant | |
| Temperatur: | 40 °C | |

**Bedingungen für MDO-Spaltung im SEG-Sammelbehälter (1)**

| | |
|---|---|
| - Verweilzeit: | 1,5 h |
| - Temperatur: | 45 °C |
| - Konzentration an Wasser: | 15 % |
| - Konzentration an Säure: | 0,2 % |
| - Abluftableitung: Absaugung des gebildeten Acetaldehyds, Abführung über Abluftsam- melleitung zur thermischen oder katalytischen Abluftbehandlung | |
| - Spaltungsrate an MDO: 50% | |

**Strom 4** (SEG-Strom + Retentat, nach MDO-Spaltung zu 50 %)

| | | |
|---|---|---|
| Massenstrom: | 1555 kg/h | |
| Ethylenglykol mit | | |
| Wasser: | 15 % | |
| 2-Methyl-1,3-dioxolan: | 0,2 % | |
| Essigsäure: | 0,2 % | *) |
| 1,4-Dioxan: | 0,065 % | |
| Acetaldehyd: | nicht relevant | |
| Temperatur: | 40 °C | |

**EG-Rückgewinnungsrate**

| |
|---|
| Zusätzlich zur Prozesskolonne geführtes freies EG: 10,1 kg/h = 0,73 kg/t PET |

Das bei der MDO-Spaltung neben dem Ethylenglykol gebildete Acetaldehyd wird zum größten Teil über die Entlüftung des SEG-Sammelbehälters via Abluftsammelleitung einer thermischen oder katalytischen Abluftreinigung zugeführt.

### Beispiel 2

Die folgenden Tabellen zeigen die Stoffströme in einem PET-Herstellungsprozess, wobei im Unterschied zum Verfahren nach Beispiel 1 das teilgereinigte Prozessabwasser nach Durchlaufen von Prozesskolonne (5) und Kondensator (6) nicht gestrippt wird.
**Strom 16** (Teilgereinigtes Prozessabwasser nach Prozesskolonne und Kühler)

| | | |
|---|---|---|
| Massenstrom: | 2613 kg/h | |
| Wasser mit | | |
| Ethylenglykol: | 0,5 % | |
| 2-Methyl-1,3-dioxolan: | 0,45 % | |
| Essigsäure: | 0,18 % | *) |
| 1,4-Dioxan: | 0,05 % | |
| Acetaldehyd: | nicht relevant | |
| Temperatur: | 35 - 45 °C | |

**Strom 14** (Teilgereinigtes Prozessabwassers: Retentat aus Umkehrosmose)

| | | |
|---|---|---|
| Massenstrom: | 180 kg/h | |
| Wasser mit | | |
| Ethylenglykol: | 5,8 % | |
| 2-Methyl-1,3-dioxolan: | 5,5 % | |
| Essigsäure: | 1,3 % | *) |
| 1,4-Dioxan: | 0,65 % | |
| Acetaldehyd: | nicht relevant | |
| Temperatur: | 30 - 40 °C | |

**Rückhaltegrade der Umkehrosmose gegenüber:**

| | |
|---|---|
| Ethylenglykol: | 80 % |
| 2-Methyl-1,3-dioxolan: | 85 % |
| Essigsäure: | 50 % |
| 1,4-Dioxan: | 90 % |
| Acetaldehyd: | nicht relevant |

**Strom 15** (Teilgereinigtes Prozessabwasser: Permeat aus Umkehrosmose)

| | | |
|---|---|---|
| Massenstrom: | 2433 kg/h | |
| Wasser mit | | |
| Ethylenglykol: | 0,1 % | |
| 2-Methyl-1,3-dioxolan: | 0,07 % | |
| Essigsäure: | 0,095 % | *) |
| 1,4-Dioxan: | 0,004 % | |
| Acetaldehyd: | nicht relevant | |
| Temperatur: | 30 - 40 °C | |
| Ableitung als Abwasser zur Endreinigung in einer chemisch-biologischen Abwasserreinigung | | |

**Strom 2** (SEG von Vorpolykondensation)

| | | |
|---|---|---|
| Massenstrom: | 1375 kg/h | |
| Ethylenglykol mit | | |
| Wasser: | 5 % | |
| 2-Methyl-1,3-dioxolan: | 0,45 % | |
| Essigsäure: | 0,1 % | *) |
| 1,4-Dioxan: | 0,03 % | |
| Acetaldehyd: | nicht relevant | |
| Temperatur: | 40 °C | |

**Bedingungen für MDO-Spaltung im SEG-Sammelbehälter (1)**

| | |
|---|---|
| - Verweilzeit: | 1,5 h |
| - Temperatur: | 45 °C |
| - Konzentration an Wasser: | 15 % |
| - Konzentration an Säure: | 0,2 % |
| - Abluftableitung: Absaugung des gebildeten Acetaldehyds, Abführung über Abluftsammelleitung zur thermischen oder katalytischen Abluftbehandlung | |
| - Spaltungsrate an MDO: 50% | |

**Strom 4** (SEG-Strom + Retentat, nach MDO-Spaltung zu 50 %)

| | | |
|---|---|---|
| Massenstrom: | 1555 kg/h | |
| Ethylenglykol mit | | |
| Wasser: | 14 % | |
| 2-Methyl-1,3-dioxolan: | 0,5 % | |
| Essigsäure: | 0,24 % | *) |
| 1,4-Dioxan: | 0,1 % | |
| Acetaldehyd: | nicht relevant | |
| Temperatur: | 40 °C | |

**EG-Rückgewinnungsrate**

| |
|---|
| Zusätzlich zur Prozesskolonne geführtes freies EG: 16,2 kg/h = rd. 1,2 kg/t PET |

Das bei der MDO-Spaltung neben dem Ethylenglykol gebildete Acetaldehyd wird zum größten Teil über die Entlüftung des SEG-Sammelbehälters via Abluftsammelleitung einer thermischen oder katalytischen Abluftreinigung zugeführt.

### Bezugszeichenliste:

- 1.: Behälter / SEG-Sammelbehälter
- 2.: *(Strom 2):* SEG von Vorpolykondensation
- 3.: *(Strom 3):* Abluft von Behälter
- 4.: *(Strom 4):* SEG + Retentat aus Umkehrosmose
- 5.: Prozesskolonne
- 6.: Kondensator
- 7.: Strippersystem
- 8.: *(strom 8):* Luft
- 9.: *(Strom 9):* Gesamtabluft zur Behandlung
- 10.: *(Strom 10):* Teilgereinigtes Prozessabwasser nach Stripper
- 11.: Kühler
- 12.: Vorfilter
- 13.: Umkehrosmoseeinheit
- 14.: *(Strom 14):* Teilgereinigtes Prozessabwasser /
Retentat aus Umkehrosmose
- 15.: *(Strom 15):* Teilgereinigtes Prozessabwasser /
Permeat aus Umkehrosmose
- 16.: *(Strom 16):* Teilgereinigtes Prozessabwasser nach Prozesskolonne
(alternative Ausführungsform ohne Einsatz eines Strippers)
- 17.: Leitung (für Prozessabwasser in Form von Prozessbrüden)
- 18.: Veresterungsreaktor
- 19.: Pastenansatz
- 20.: Leitungen (für Monomere)
- 21.: (Vor-)Polykondensationsreaktor
- 22.: Leitung (für Polymer)
- 23.: Leitung (für Brüden aus der (Vor-)Polykondensation)
- 24: Vakuumeinrichtung (inkl. Kondensator)
- 25.: Leitung (für Abluft)

## Patentansprüche

1. Verfahren zur Rückgewinnung von Ethylenglykol beim PET-Herstellungsprozess, durch eine Veresterungsreaktion bei der Prozessabwasser entsteht,
- wobei das Prozessabwasser mindestens einem Trennungsschritt, einer Rektifikation, unterzogen wird, bei welchem die im Prozessabwasser vorhandenen Verunreinigungen teilweise abgetrennt werden und so ein teilgereinigtes Prozessabwasser entsteht,
- **dadurch gekennzeichnet, dass** zumindest ein Teil des teilgereinigten Prozessabwassers mit einem MDO-enthaltenden Fluid, vorzugsweise einem oder mehreren Ethylenglykol-Prozessströmen, in einem der Rektifikationskolonne vorgelagerten Behälter gemischt wird und in diesem Behälter das vorhandene MDO ganz oder teilweise in Ethylenglykol und Acetaldehyd gespalten wird, bevor die Mischung in die Rektifikationskolonne weitergeleitet wird.

2. Verfahren nach Anspruch 1, **dadurch gekennzeichnet, dass** mindestens ein Teil des teilgereinigten Prozessabwassers vor dem Mischen mit dem MDO-enthaltenden Fluid einer Umkehrosmose und/oder Ultra/Nanofiltration unterzogen wird,
- wobei vorzugsweise derjenige Teil des teilgereinigten Prozessabwassers, der bei der Umkehrosmose und/oder Ultra/Nanofiltration als Retentat anfällt, zumindest teilweise zur Mischung mit dem MDO-enthaltenden Fluid eingesetzt wird.

3. Verfahren nach einem der vorangehenden Ansprüche, **dadurch gekennzeichnet, dass** die Spaltung des MDO in Anwesenheit von einem oder mehreren Katalysatoren erfolgt,
- wobei es sich bei den Katalysatoren vorzugsweise um einen oder mehrere der folgenden handelt: "organische aliphatische und aromatische Säuren, vorzugsweise Essigsäure".

4. Verfahren nach einem der vorangehenden Ansprüche, **dadurch gekennzeichnet, dass** eine oder mehrere im Prozessabwasser enthaltene Säuren, vorzugsweise Essigsäure, mittels Umkehrosmose aufkonzentriert und als Katalysatoren für die Spaltung des MDO eingesetzt werden.

5. Verfahren nach einem der vorangehenden Ansprüche, **dadurch gekennzeichnet, dass** die Mischung einen Wassergehalt von mindestens 10 Gew.-%, vorzugsweise 13 bis 45 Gew.-%, besonders bevorzugt 15 - 25 Gew.-% aufweist.

6. Verfahren nach einem der vorangehenden Ansprüche, **dadurch gekennzeichnet, dass** zumindest ein Teil des im Prozessabwasser enthaltenen 1,4-Dioxans mittels Umkehrosmose im Prozess zurückgehalten wird.

7. Anlage zur Herstellung von PET mit
- mindestens einem Veresterungs- oder Umesterungsreaktor sowie
- mindestens einer Einrichtung zur Herstellung eines teilgereinigten Prozessabwassers durch Abtrennung von im Prozessabwasser vorhandenen Verunreinigungen,
- wobei es sich bei dieser um eine Rektifikationskolonne handelt,
**dadurch gekennzeichnet, dass** die Anlage einen der Rektifikationskolonne vorgelagerten Behälter aufweist
in welchem vorhandenes MDO ganz oder teilweise in Ethylenglykol und Acetaldehyd gespalten wird, bevor die Mischung in die Rektifikationskolonne weitergeleitet wird und welcher
- eine oder mehrere Zuleitungen für das teilgereinigte Prozessabwasser und
- eine oder mehrere Zuleitungen für ein MDO-enthaltendes Fluid, vorzugsweise einen Ethylenglykol-Prozeßstrom,
- und/oder eine oder mehrere Zuleitungen für eine Mischung aus teilgereinigtem Prozessabwasser und einem MDO-enthaltenden Fluid aufweist.

8. Anlage nach Anspruch 7, **dadurch gekennzeichnet, dass** die Anlage eine Umkehrosmoseeinrichtung und/oder Ultra/Nanofiltrationseinrichtung aufweist, welche vor dem Behälter angeordnet ist.

9. Anlage nach Anspruch 7 oder 8, **dadurch gekennzeichnet, dass** das Volumen des Behälters so bemessen ist, dass die Verweilzeit einer Mischung beinhaltend das teilgereinigte Prozessabwasser und das MDO-enthaltende Fluid 0,2 - 4 Stunden, vorzugsweise 0,5 - 3 Stunden, besonders bevorzugt 1 - 2 Stunden beträgt.

## Claims

1. A method of recovering ethylene glycol in the PET production process through an esterification reaction with the process wastewater occurs,
- wherein the process wastewater undergoes at least a separation step, rectification, in which some of the impurities present in the process wastewater are separated and partially purified process wastewater is thereby produced,
- **characterised in that** at least part of the partially purified process wastewater is mixed with an MDO-containing fluid, preferably one or several ethylene glycol process streams, in a vessel upstream of the rectification column and in this vessel the MDO present is completely or partially split into ethylene glycol and acetaldehyde before the mixture is transferred into the rectification column.

2. The method according to claim 1, **characterised in that** at least part of the partially purified process wastewater undergoes reverse osmosis and/or ultra/nanofiltration prior to mixing with the MOD-containing fluid,
- wherein that part of the partially purified process wastewater produced as retentate during reverse osmosis and/or ultra/nanofiltration is preferably used at least in part to mix with the MDO-containing fluid.

3. The method according to one of the preceding claims, **characterised in that** the splitting of the MDO takes place in the presence of one or several catalysts,
- wherein the catalysts are preferably one or several of the following: "organic aliphatic and aromatic acids, preferably acetic acid".

4. The method according to one of the preceding claims, **characterised in that** one or several acids contained in the process wastewater, preferably acetic acid, are concentrated by means of reverse osmosis and used as catalysts for the splitting of the MDO.

5. The method according to one of the preceding claims, **characterised in that** the mixture exhibits a water content of at least 10 % by weight, preferably 13 to 45 % by wt., particularly preferably, 15-25 % by wt.

6. The method according to one of the preceding claims, **characterised in that** at least part of the 1,4-dioxane contained in the process wastewater is retained in the process by means of reverse osmosis.

7. Plant for producing PET with
- at least one esterification or transesterification reactor and
- at least one facility for producing partially purified process wastewater by separating impurities present in the process wastewater,
- wherein this preferably involves a rectification column,
**characterised in that** the plant has a vessel upstream of the rectification column
in which MDO that is present is completely or partially split into ethylene glycol and acetaldehyde, before the mixture is transferred into the rectification column and which exhibits
- one or several supply lines for the partially purified process wastewater and
- one or several supply lines for an MDO-containing fluid, preferably an ethylene glycol process stream,
- and/or one or several supply streams for a mixture of partially purified process wastewater and an MDO-containing fluid.

8. The plant according to claim 7, **characterised in that** the plant exhibits a reverse osmosis device and/or ultra/nanofiltration facility, which is disposed upstream of the container.

9. The plant according to claim 7 or 8, **characterised in that** the vessel volume is of such dimensions that the dwell time of a mixture containing the partially purified process water and the MDO-containing fluid is 0.2-4 hours, preferably 0.5-3 hours, particularly preferably 1-2 hours.

## Revendications

1. Procédé de récupération d'éthylène glycol lors d'un processus de fabrication de PET, par une réaction d'estérification dans les eaux résiduaires du processus,
- les eaux résiduaires du processus étant soumises au moins à une étape de séparation, à une rectification, lors de laquelle les impuretés présentes dans les eaux résiduaires du processus sont séparées pour obtenir des eaux résiduaires du processus partiellement purifiées,
- **caractérisé en ce qu'**au moins une partie des eaux résiduaires du processus partiellement purifiées est mélangée à un fluide contenant du MDO, de préférence à un ou à plusieurs flux d'éthylène glycol du processus dans un réservoir monté en amont de la colonne de rectification et dans ce réservoir, le MDO présent est craqué totalement ou en partie en éthylène glycol et en aldéhyde éthylique, avant que le mélange ne soit conduit dans la colonne de rectification.

2. Procédé selon la revendication 1, **caractérisé en ce qu'**avant d'être mélangée au fluide contenant du MDO, au moins une partie des eaux résiduaires du processus partiellement purifiées est soumise à une osmose inverse et/ou à une ultrafiltration/nanofiltration,
- ladite partie des eaux résiduaires du processus partiellement purifiées, qui se produit en tant que rétantat lors de l'osmose inverse et/ou de l'ultrafiltration/de la nanofiltration étant mise en oeuvre au moins en partie pour être mélangée au fluide contenant du MDO.

3. Procédé selon l'une quelconque des revendications précédentes, **caractérisé en ce que** le craquage du MDO s'effectue en présence d'un ou de plusieurs catalyseurs,
- s'agissant pour le catalyseur, de préférence de l'un ou de plusieurs de ceux qui sont cités ci-après : « acides aliphatiques et aromatiques organiques, de préférence acide acétique ».

4. Procédé selon l'une quelconque des revendications précédentes, **caractérisé en ce qu'**un ou plusieurs acides contenus dans les eaux résiduaires du processus, de préférence de l'acide acétique sont concentrés par osmose inverse et mis en oeuvre en tant que catalyseurs pour le craquage du MDO.

5. Procédé selon l'une quelconque des revendications précédentes, **caractérisé en ce que** le mélange présente une teneur en eau d'au moins 10 % en poids, de préférence de 13 à 45 % en poids, de manière particulièrement préférée de 15 à 25 % en poids.

6. Procédé selon l'une quelconque des revendications précédentes, **caractérisé en ce qu'**au moins une partie du 1,4-dioxane contenu dans les eaux résiduaires du processus est retenue par osmose inverse.

7. Installation de production de PET, avec
- au moins un réacteur d'estérification ou de transestérification, ainsi
- qu'au moins un dispositif de production d'eaux résiduaires du processus partiellement purifiées par séparation des impuretés présentes dans les eaux résiduaires du processus,
- s'agissant de préférence pour ce dernier d'une colonne de rectification,
**caractérisée en ce que** l'installation présente un réservoir monté en amont de la colonne de rectification
dans lequel le MDO présent est craqué totalement ou en partie en éthylène glycol et en aldéhyde éthylique, avant que le mélange ne soit conduit dans la colonne de rectification et lequel
- comporte un ou plusieurs conduits d'alimentation des eaux résiduaires du processus en partie purifiées et
- un ou plusieurs conduits d'alimentation d'un fluide contenant du MDO, de préférence un flux de processus d'éthylène glycol,
- et/ou un ou plusieurs conduits d'alimentation d'un mélange d'eaux résiduaires du processus partiellement purifiées et d'un fluide contenant du MDO.

8. Installation selon la revendication 7, **caractérisée en ce que** l'installation comporte un dispositif d'osmose inverse et/ou un dispositif d'ultrafiltration/de nanofiltration, lequel est disposé en amont du réservoir.

9. Installation selon la revendication 7 ou 8, **caractérisé en ce que** le volume du réservoir est dimensionné de sorte que le temps de séjour d'un mélange contenant les eaux résiduaires du processus partiellement purifiées et le fluide contenant du MDO soit de 0,2 à 4 heures, de préférence de 0,5 à 3 heures, de manière particulièrement préférée de 1 à 2 heures.
